# FASCICULE DE BREVET EUROPEEN

(11) **EP 1 768 988 B1**
(45) Date de publication et mention de la délivrance du brevet: **28.01.2009**
(21) Numéro de dépôt: 04805689.9
(22) Date de dépôt: 10.12.2004
(51) Int. Cl.: C07F 5/02, C07C 401/00

(54) **NOUVEAUX DERIVES D'ACIDES PHENYL-BORONIQUES ET LEURS PROCEDES DE PREPARATION**
NEUE PHENYLBORONSÄUREDERIVATE UND VERFAHREN ZU DEREN HERSTELLUNG
NOVEL PHENYL-BORONIC ACID DERIVATIVES AND METHODS FOR THE PREPARATION THEREOF

(30) Priorité: 18.12.2003 FR 0314946
(43) Date de publication de la demande: 04.04.2007
(73) Titulaire: GALDERMA RESEARCH & DEVELOPMENT, 06410 Biot (FR)
(72) Inventeur: TERRANOVA, Eric, F-06520 Magagnosc (FR); PASCAL, Jean-Claude, F-06100 Nice (FR)
(74) Mandataire: Allab, Myriam
(86) Numéro de dépôt international: PCT/FR2004/003192
(87) Numéro de publication internationale: WO 2005/061520

(56) Documents cités:
- WO-A-03/050067
- US-A1- 2002 143 024

## Description

La présente invention concerne de nouveaux dérivés disubstitués de l'acide phényl-boronique de formule générale (I) : 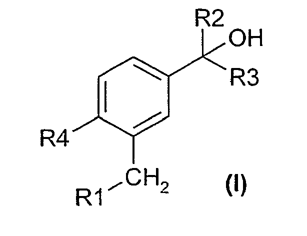 dans laquelle :
- R1 représente un atome d'hydrogène ou un radical alkyle de 1 à 4 atomes de carbone,
- R2 et R3, identiques ou différents représentent un groupement alkyle de 1 à 4 atomes de carbone,
- R4 représente le radical -B(OH)₂ ou le radical de formule : 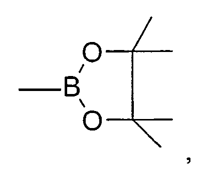
leurs procédés de préparation et leur utilisation en tant qu'intermédiaires dans la synthèse de dérivés non stéroïdiens de vitamine D.

Les dérivés de l'acide phényl-boronique peuvent être utilisés dans des réactions de couplage de type Suzuki et sont des intermédiaires importants dans la synthèse d'analogues non stéroïdiens de la Vitamine D3. De telles réactions ont été décrites par la demanderesse dans la demande de brevet WO 03/050067.

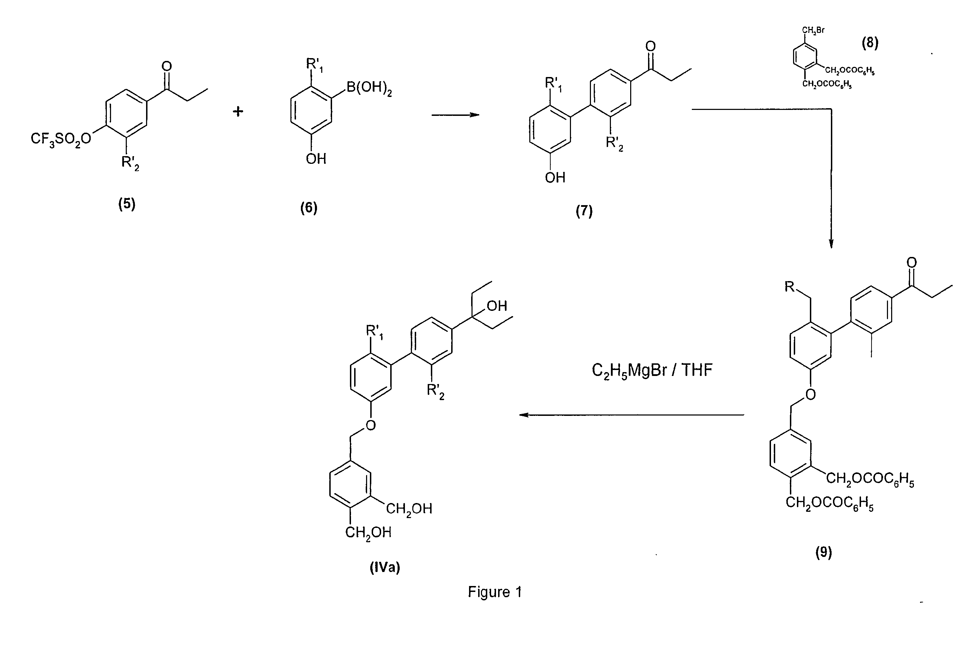

Dans la demande de brevet WO 03/050067, les dérivés non stéroïdiens de vitamine D de formule générale (IVa) (voir la **figure 1**) sont obtenus à partir de triflates intermédiaires de formule générale (5) et d'acides boroniques intermédiaires de formule générale (6).

La préparation des triflates intermédiaires de formule générale (5) nécessite 3 étapes de synthèse et celle des acides boroniques intermédiaires de formule générale (6) nécessite 6 étapes de synthèses.

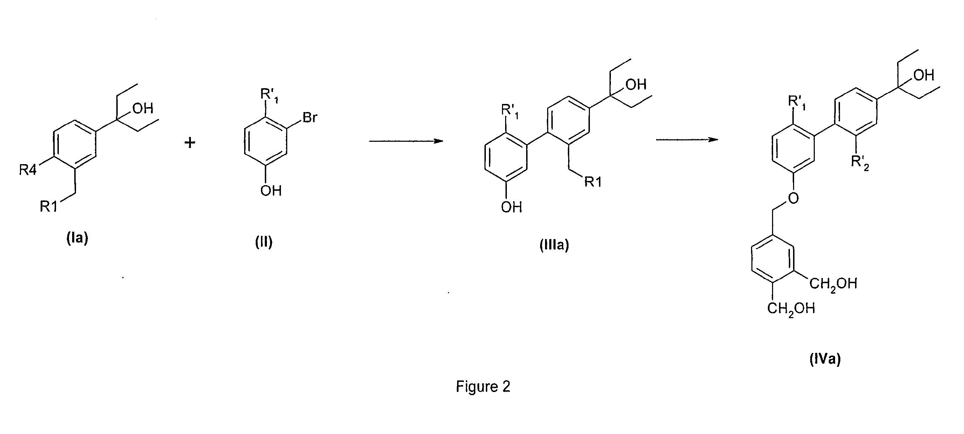

Dans la présente invention, la synthèse des acides phényl-boroniques de formule générale (la) se fait en 4 étapes et nécessite l'utilisation de dérivés bromés de formule générale (II) pour accéder aux dérivés non stéroïdiens de vitamine D de formule générale (IVa) selon la **figure 2**. Les dérivés bromés de formule générale (II) sont préparés en 3 étapes.

L'utilisation des acides phényl-boroniques de formule générale (I) de la présente invention présente donc comme premier avantage d'accéder aux intermédiaires biphényles de formule générale (III) en 7 étapes au total (voir figure 2) alors que la synthèse décrite précédemment dans la demande de brevet WO 03/050067 nécessite 9 étapes pour accéder aux intermédiaires biphényles de formule générale (7) (figure 1).

L'utilisation des acides phényl-boroniques de formule générale (I) nécessite l'emploi de dérivés bromés de formule générale (II), ce qui présente comme deuxième avantage d'éviter les étapes de protection et de déprotection nécessaires pour préparer les acides boroniques intermédiaires de formule générale (6) utilisés dans la précédente synthèse (figure 1). De plus, l'étape de protection était réalisée avec du chlorure de méthoxyméthyle, réactif hautement cancérigène et interdit à l'échelle industrielle.

Un troisième avantage apporté par l'utilisation des acides phényl-boroniques de formule générale (I) est qu'ils permettent d'accéder aux intermédiaires biphényles de formule générale (III) dans lesquels la fonction alcool tertiaire est déjà présente alors qu'en passant par les intermédiares biphényles de formule générale (7) tels que décrits dans la demande de brevet WO 03/050067 (figure 1), il faut encore transformer la fonction cétone en fonction alcool tertiaire.

Un quatrième avantage apporté par l'utilisation des acides phényl-boroniques de formule générale (I) est qu'ils permettent d'accéder aux dérivés non stéroïdiens de vitamine D de formule générale (IVa) en moins d'étapes qu'avec la synthèse telle que décrite dans la demande de brevet WO 03/050067 et avec un meilleur rendement.

Dans la présente invention, la demanderesse a donc réalisé la synthèse de nouveaux dérivés d'acides phényl-boroniques et mis au point un nouveau procédé d'obtention permettant de remédier aux problèmes exposés ci-dessus.

La présente invention concerne de nouveaux dérivés disubstitués de l'acide phényl-boronique de formule générale (I) : 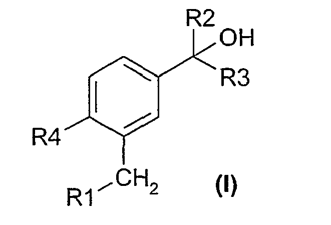 dans laquelle :
- R1 représente un atome d'hydrogène ou un radical alkyle de 1 à 4 atomes de carbone,
- R2 et R3, identiques ou différents représentent un groupement alkyle de 1 à 4 atomes de carbone,
- R4 représente le radical -B(OH)₂ ou le radical de formule :

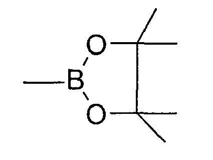

Selon la présente invention, par radical alkyle ayant de 1 à 4 atomes de carbone, on entend un radical contenant 1 à 4 atomes de carbone, saturé ou insaturé, linéaire ou cyclique, éventuellement ramifié, pouvant être interrompu par un hétéroatome, et de préférence, les radicaux alkyle ayant de 1 à 4 atomes de carbone sont les radicaux méthyle, éthyle, propyle, isopropyle, cyclopropyle, butyle, isobutyle ou tertiobutyle.

La présente invention se rapporte également aux nouveaux composés de formules (2) et (3) suivants : 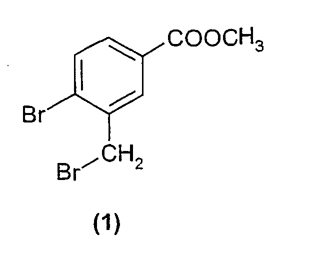 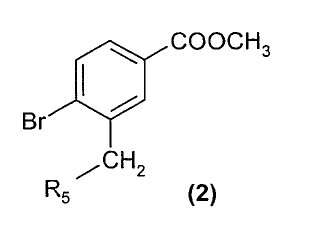 et 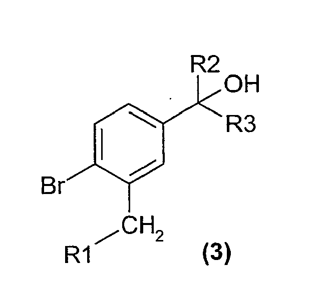 avec, pour les composés (2) et (3), R1 représente un atome d'hydrogène ou un radical alkyle de 1 à 4 atomes de carbone, R2 et R3, identiques ou différents représentent un groupement alkyle de 1 à 4 atomes de carbone et R5 représente un radical alkyle de 1 à 4 atomes de carbone.

L'invention se rapporte également à l'utilisation des composés (1), (2) et (3) pour la préparation d'analogues non stéroidiens de vitamine D, et, en particulier, comme intermédiaires de synthèse pour la préparation des composés de formule (I) selon l'invention.

L'invention concerne donc également un procédé de synthèse de ces nouveaux dérivés d'acides phényl-boroniques de formule (I).

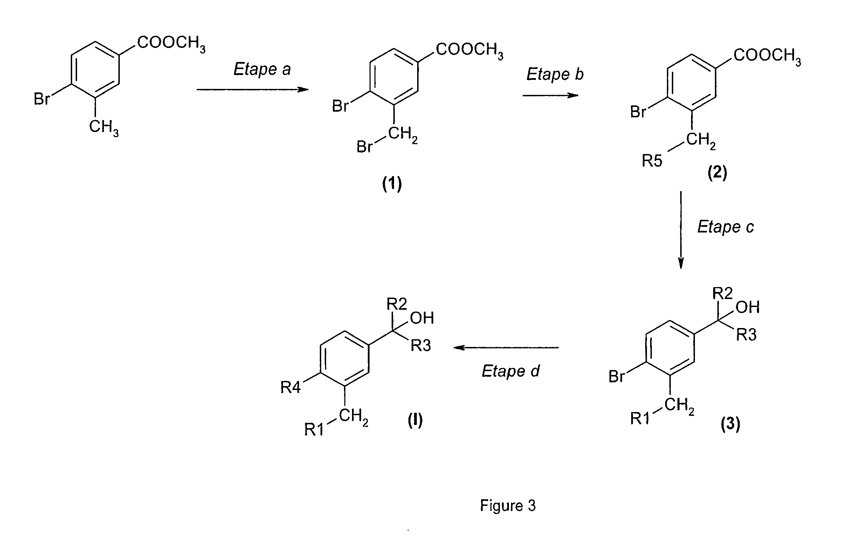

La synthèse de ces nouveaux dérivés d'acide phényl-boronique de formule générale (I) dans laquelle R4 représente le radical B(OH)₂ se fait en 4 étapes à partir du 4-bromo-3-methyl benzoate de méthyle (commercial) selon le schéma de la **figure 3**.

Ainsi, le procédé de préparation des composés de formule générale (I) dans laquelle R4 représente le radical B(OH)₂ comporte les étapes suivantes, les étape a) et b) n'étant pas mises en oeuvre dans le cas particulier où R1 est un atome d'hydrogène :
a) Bromation en position benzylique en présence d'un solvant pour obtenir le composé (1) ; 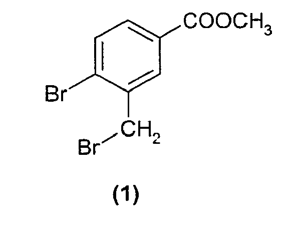
b) Substitution du brome introduit à l'étape (a) en position benzylique par une chaîne alkyle R5 comportant 1 à 4 atomes de carbones, pour obtenir le composé (2) tel que défini précédemment : 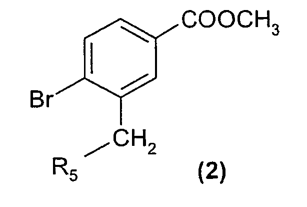
c) Transformation de la fonction ester des intermédiaires de formule générale (2) ou du 4-bromo-3-methyl benzoate de méthyle en fonction alcool tertiaire en utilisant au moins deux équivalents d'un organomagnésien de formule R2-MgX
   avec X représente un atome de Chlore ou de Brome
   et R2 représente un radical méthyle, éthyle, propyle ou butyle pour conduire au composé de formule générale (3) tel que défini précédemment ; 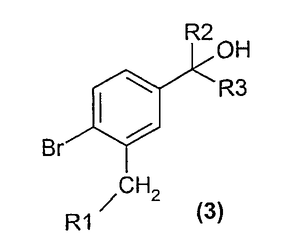
d) Transformation du bromure aromatique des intermédiaires de formule générale (3) en acide phényl-boronique de formule (I) en présence d'un solvant, d'une base et d'un trialkyle borate.

De façon plus détaillée, les étapes de ce procédé peuvent être mises en oeuvre de la façon suivante :
a. La première étape consiste à effectuer une bromation en position benzylique pour obtenir l'intermédiaire (1). Cette réaction d'halogénation est documentée dans la littérature. On peut utiliser différents agents de bromation comme par exemple le brome (K. Smith & col/., J. Chem. Soc. Perkin. Trans. I, 2000, 2745), la N-bromosuccinimide (P. Liu & coll., Synthesis, 2001, 2078*),* le bromate de sodium (D. Kikuchi & coll., J. Org. Chem.,1998, 6023) ou encore la 1,3-dibromo-5,5-dimethylhydantoïne *(*H. Jendralla & coll., Liebigs Ann. Chem., 1995, 1253). Cette réaction s'effectue le plus souvent dans des solvants chlorés ou dans des éthers. L'agent de bromation utilisé préférentiellement est la N-bromosuccunimide au reflux du dichlorométhane. De préférence, on utilise également du péroxyde de benzoyle en quantité catalytique comme initiateur radicalaire de la réaction. Pour activer la réaction radicalaire, on irradie le milieu réactionnel avec une lampe de 1000 watts.
   Cette étape conduit à l'intermédiaire (1). 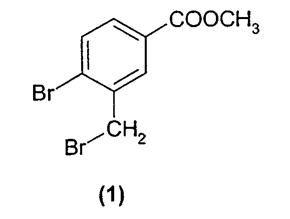
b. La seconde étape consiste à substituer le brome introduit à l'étape 1 en position benzylique par une chaîne alkyle R'1 comportant 1 à 4 atomes de carbones. Pour favoriser cette réaction de substitution, on accroît le caractère nucléophile du groupe alkyle R'1 en utilisant un réactif organométallique R'1-M dans lequel M représente un atome de métal comme le magnésium, le cuivre ou encore le zinc. Cette réaction est documentée dans la littérature (B.H. Lipshutz and S. Sengupta, Organic Reactions, 1992, 41, 135)*.* De préférence, on utilise du cuivre sous forme d'iodure de cuivre en présence d'un organomagnésien R'1-MgX (X=Br, CI, I) pour générer un organocuprate. Cette réaction peut s'effectuer dans des solvants éthérés. De préférence, le solvant éthéré utilisé est le tétrahydrofuranne. La réaction est effectuée à basse température, de préférence entre -40°C et 0°C, plus particulièrement, entre -40°C et -20°C.
   Cette étape conduit aux intermédiaires de formule générale (2) dont les radicaux sont définis précédemment. 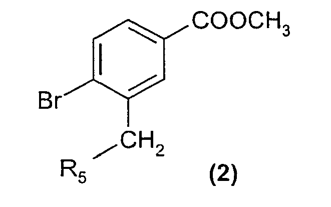
c. La troisième étape consiste à transformer la fonction ester des intermédiaires de formule générale (2) ou du 4-bromo-3-methyl benzoate de méthyle en fonction alcool tertiaire en utilisant au moins deux équivalents d'un organomagnésien de formule R2-MgX avec X représente un atome de chlore, de brome ou d'iode et R2 représente un radical alkyle ayant de 1 à 4 atomes de carbone. Cette réaction est largement documentée dans la littérature. Cette réaction peut s'effectuer dans des solvants éthérés. De préférence, le solvant éthéré utilisé est le tétrahydrofuranne. La réaction est effectuée de préférence à une température comprise entre -20°C et +20°C, et plus particulièrement, entre -10°C et +10°C.
   Cette étape conduit à aux intermédiaires de formule générale (3) dont les radicaux sont définis précédemment. 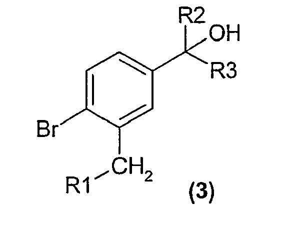
d. La quatrième étape consiste à transformer le bromure aromatique des intermédiaires de formule générale (3) en acide phényl-boronique de formule (I). Cette réaction, bien documentée dans la littérature (N. Miyaura & A. Suzuki, Chem. Rev., 1995, 95, 2457)*,* s'effectue dans le tetrahydrofuranne à des températures comprises entre -78°C et -20°C, de préférence entre -78°C et -40°C. On utilise entre 2 et 4 équivalents d'une base forte comme le butyllithium, de préférence entre 2,5 et 3,5 équivalents et un trialkyle borate comme le trimethyl borate ou le triisopropyl borate. On utilise entre 2 et 4 équivalents de ce dernier, de préférence entre 2,5 et 3,5 équivalents.

Selon un autre mode selon l'invention, la synthèse de ces nouveaux dérivés d'acide phényl-boronique de formule générale (I) dans laquelle R4 représente le radical de formule : 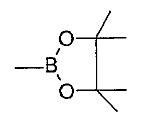 se fait en 4 étapes à partir du 4-bromo-3-methyl benzoate de méthyle (commercial) selon le schéma de la **figure 3**.

Les trois premières étapes sont similaires au procédé décrit ci-dessus.

Ainsi, le procédé de préparation des composés de formule générale (I) dans laquelle R4 représente le radical 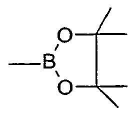 comporte les étapes suivantes, les étapes a) et b) n'étant pas mises en oeuvre dans le cas où R1 est un atome d'hydrogène :
a) Bromation en position benzylique en présence d'un solvant pour obtenir le composé (1) ; 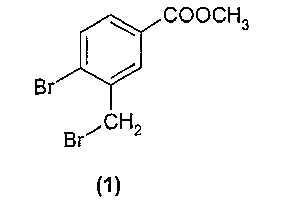
b) Substitution du brome introduit à l'étape (a) en position benzylique par une chaîne alkyle R5 comportant 1 à 4 atomes de carbones, pour obtenir le composé de formule générale (2) tel que défini ci-dessus : 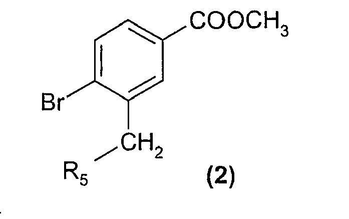
c) Transformation de la fonction ester des intermédiaires de formule générale (2) ou du 4-bromo-3-methyl benzoate de méthyle en fonction alcool tertiaire en utilisant au moins deux équivalents d'un organomagnésien de formule R2-MgX
   avec X représente un atome de Chlore ou de Brome
   et R2 représente un radical méthyle, éthyle, propyle, butyle ou isobutyle pour conduire au composé de formule générale (3) tel que défini ci-dessus 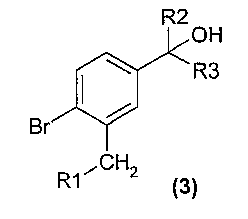
d) Transformation du bromure aromatique en aryl boronate en présence d'un solvant, d'un catalyseur, d'une base et de pinacol borane ou de pinacoldiborane pour obtenir le composé de formule générale (I).

De façon plus détaillée, les étapes de ce procédé peuvent être mises en oeuvre de la façon suivante :
a. La première étape consiste donc à effectuer une bromation en position benzylique pour obtenir l'intermédiaire (1). Cette réaction d'halogénation est documentée dans la littérature. On peut utiliser différents agents de bromation comme par exemple le brome (K. Smith & coll., J. Chem. Soc. Perkin. Trans. I, 2000, 2745), la N-bromosuccinimide (P. Liu & coll., Synthesis, 2001, 2078)*,* le bromate de sodium (D. Kikuchi & coll., J. Org. Chem.,1998, 6023) ou encore la 1,3-dibromo-5,5-dimethylhydantoïne (H. Jendralla & coll., Liebigs Ann. Chem., 1995, 1253)*.* Cette réaction s'effectue le plus souvent dans des solvants chlorés ou dans des éthers. L'agent de bromation utilisé préférentiellement est la N-bromosuccunimide au reflux du dichlorométhane. De préférence, on utilise également du péroxyde de benzoyle en quantité catalytique comme initiateur radicalaire de la réaction. Pour activer la réaction radicalaire, on irradie le milieu réactionnel avec une lampe de 1000 watts.
   Cette étape conduit à l'intermédiaire (1). 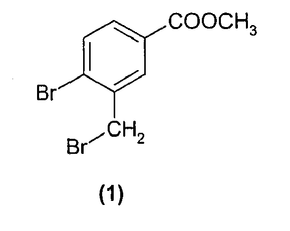
b. La seconde étape consiste à substituer le brome introduit à l'étape 1 en position benzylique par une chaîne alkyle R'1 comportant 1 à 4 atomes de carbones. Pour favoriser cette réaction de substitution, on accroît le caractère nucléophile du groupe alkyle R'1 en utilisant un réactif organométallique R'1-M dans lequel M représente un atome de métal comme le magnésium, le cuivre ou encore le zinc. Cette réaction est documentée dans la littérature (B.H. Lipshutz and S. Sengupta, Organic Reactions, 1992, 41, 135). De préférence, on utilise du cuivre sous forme d'iodure de cuivre en présence d'un organomagnésien R'1-MgX (X=Br, CI, I) pour générer un organocuprate. Cette réaction peut s'effectuer dans des solvants éthérés. De préférence, le solvant éthéré utilisé est le tétrahydrofuranne. La réaction est effectuée à basse température, de préférence entre -40°C et 0°C, plus particulièrement, entre -40°C et -20°C.
   Cette étape conduit aux intermédiaires de formule générale (2) dont les radicaux sont définis précédemment. 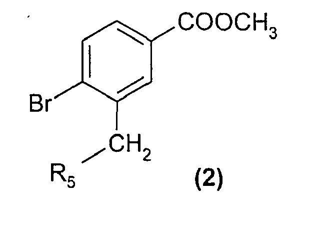
c. La troisième étape consiste à transformer la fonction ester des intermédiaires de formule générale (2) ou du 4-bromo-3-methyl benzoate de méthyle en fonction alcool tertiaire en utilisant au moins deux équivalents d'un organomagnésien de formule R2-MgX avec X représente un atome de chlore, de brome ou d'iode et R2 représente un radical alkyle ayant de 1 à 4 atomes de carbone. Cette réaction est largement documentée dans la littérature. Cette réaction peut s'effectuer dans des solvants éthérés. De préférence, le solvant éthéré utilisé est le tétrahydrofuranne. La réaction est effectuée de préférence à une température comprise entre -20°C et +20°C, et plus particulièrement, entre -10°C et +10°C.
   Cette étape conduit à aux intermédiaires de formule générale (3) dont les radicaux sont définis précédemment. 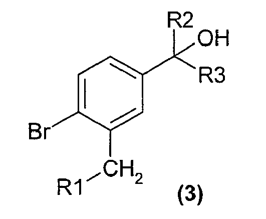
d. Lors de cette quatrième étape, on peut transformer le bromure aromatique des intermédiaires de formule générale (3) en aryl boronate. Cette réaction, également bien documentée dans la littérature (a/ M. Murata & coll ; J.Org.Chem., 1997, 62, 6458 ; *b*/ T. Ishiyama & coll., J.Org.Chem., 1995, 60, 7508), s'effectue dans des solvants comme le DMF, le tetrahydrofuranne, le DMSO ou encore le dioxanne. Les températures de réaction utilisées sont généralement proches des températures de reflux des solvants. On utilise généralement un catalyseur au palladium, préférentiellement le dichloro [1,1'-ferrocenylbis(diphenylphosphane)] palladium (II) dichloromethane ou Pd(dppf) Cl₂, une base, préférentiellement l'acétate de potassium ou la triethylamine, de préférence entre 2,5 et 3,5 équivalents et un tetra alkoxyde de bore, préférentiellement le pinacol-borane ou le pinacol-diborane. On utilise entre 1 et 1,5 équivalents de ce dernier, de préférence entre 1,0 et 1,2 équivalents.

La présente invention concerne également l'utilisation des dérivés disubstitués de l'acide phényl-boronique de formule (I) et les composés (1), (2) et (3) comme intermédiaire de synthèse, pour la préparation de dérivés de vitamine D.

Préférentiellement, les composés (I), (1), (2) et (3) selon l'invention sont utilisés comme intermédiaire de synthèse de dérivés non stéroidiens de vitamine D.

Plus préférentiellement les dérivés non stéroidiens de vitamine D sont des composés de formule (IV) : 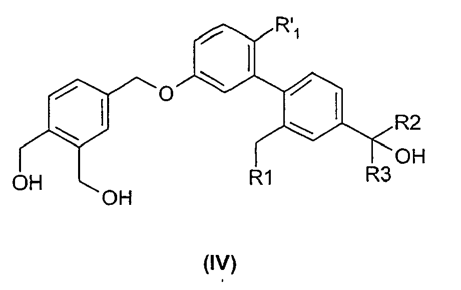 dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones.

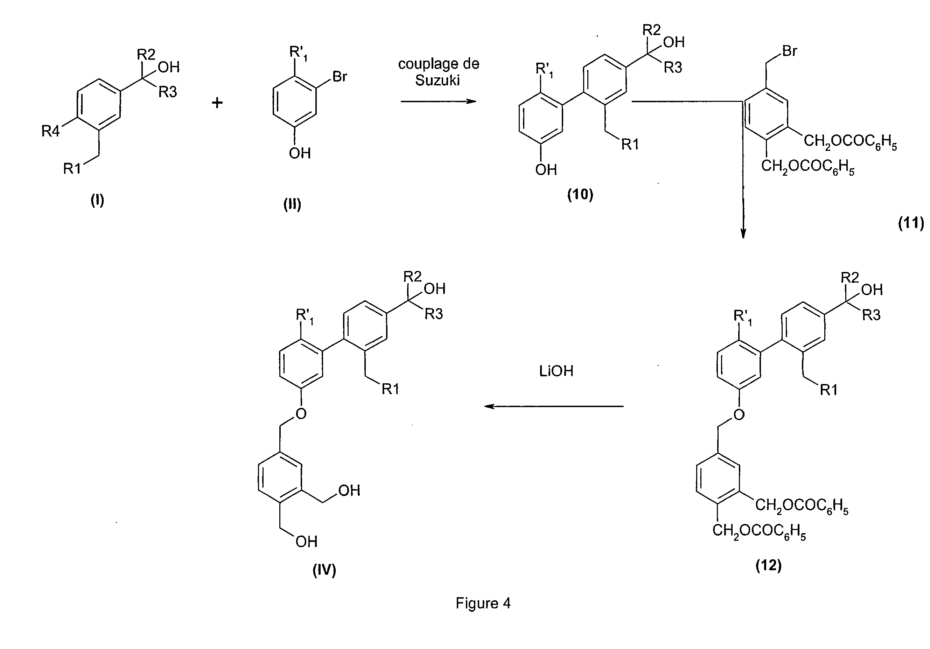

Dans le cas préféré selon l'invention, les composés selon l'invention interviennent donc dans la synthèse selon le schéma indiqué à la **figure 4**.

Selon l'invention, les composés de formule (IV) sont préparés de préférence à partir du composé (12) obtenu selon le schéma réactionnel de la figure 4. Le composé (12) peut être obtenu à partir des composés dérivés de l'acide phényl-boronique de formule générale (I) selon l'invention. Une première réaction est une réaction de couplage de type Suzuki entre les dérivés de formule générale (I) selon l'invention et un bromure d'aryle de formule générale (II). Le produit de la réaction de Suzuki, de formule générale (10), est condensé sur le bromure benzylique (11) en présence d'une base comme par exemple le carbonate de potassium pour conduire aux composés de formule générale (12). Par la suite, on met en oeuvre une réaction selon des modalité bien connues de l'homme du métier, par exemple et de façon non limitative tel que décrit dans la demande de brevet WO 03/050067, permettant, à partir du composé (12) d'obtenir le composé (IV).

La présente invention concerne donc le procédé de préparation des composés de formule (IV) à partir des acides phényl-boroniques de la présente invention selon les étapes suivantes :
a) Réaction de couplage de type Suzuki entre les composés de formule générale (I) 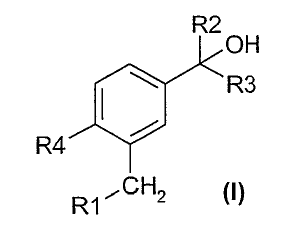 dans laquelle :
   - R1 représente un atome d'hydrogène ou un radical alkyle de 1 à 4 atomes de carbone,
   - R2 et R3, identiques ou différents représentent un radical alkyle de 1 à 4 atomes de carbone,
   - R4 représente le radical -B(OH)₂ ou le radical de formule : 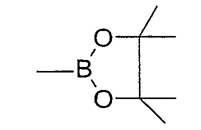 et un bromure d'aryle de formule générale (II) : 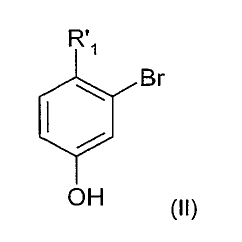 dans laquelle :
   - R'₁ représente un radical alkyle de 1 à 4 atomes de carbones,
   pour obtenir le produit de formule générale (10) : 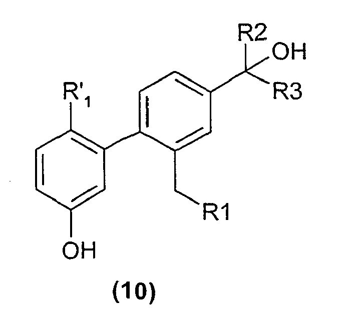 dans laquelle :
   - R₁ représente un atome d'hydrogène ou un radical alkyle de 1 à 4 atomes de carbone
   - R₂ et R₃ identiques ou différents représentent un radical alkyle de 1 à 4 atomes de carbone
   - R'₁ représente un radical alkyle de 1 à 4 atomes de carbones ;
b) Le produit de la réaction de Suzuki, de formule générale (10), est condensé sur le bromure benzylique (11) en présence d'une base, tel le carbonate de potassium ; 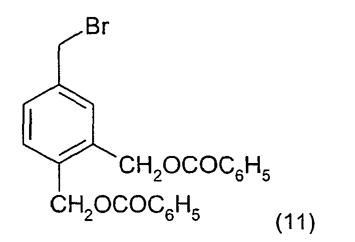
c) Libération des fonctions alcool du composé (11) pour conduire au composé (IV).

La présente invention concerne également les composés de formule (10), intermédiaires dans le procédé défini précédemment, de formule suivante : 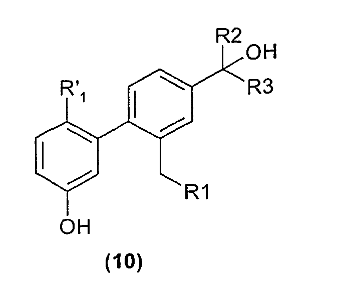 dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones ;
et leur utilisation dans la synthèse d'analogues de vitamine D tel que décrit précédemment et à la figure 2.

Sans aucun caractère limitatif, on trouvera ci-dessous des exemples de préparation des acides phényl-boroniques selon l'invention.

### Exemples :

### Exemple 1 :

### Etape 1 : 4-bromo-3-bromomethyl benzoate de méthyle

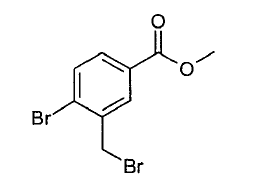

Dans un réacteur de 6 litres, équipé d'un réfrigérant, d'une agitation mécanique centrale et d'un thermomètre, introduire 575 g (2,5 moles) de 4-bromo-3-methyl benzoate de méthyle, 3 litres de chlorure de méthylène, 494 g (2,75 moles) de N-bromosuccinimide et 17 g de peroxyde de benzoyle (0,05 moles ; 70% dans l'eau).

Porter au reflux sous irradiation d'une lampe de 1000 watts pendant 10h. Laver deux fois le milieu avec 1 litre d'eau, puis avec 1 litre d'une solution saturée en chlorure de sodium. Evaporer le chlorure de méthylène. Recristalliser le produit brut obtenu dans de l'heptane. On obtient 596 g de 4-bromo-3-bromomethyl benzoate de méthyle sous forme de cristaux blanc cassé. (Rdt=77%)
Point de fusion = 100-101 °C
RMN 1 H (CDCl3 ; 400MHz) : 3,94 (s ; 3H) ; 4,64 (s ; 2H) ; 7,68 (d ; 1 H ; Jo=8,3Hz) ; 7,83 (d x d ; 1 H ; Jo=8,3Hz et Jm=2,0Hz) ; 8,14 (d ; 1 H ; Jm=2,0Hz)

### Etape 2 : 4-bromo-3-propyl benzoate de méthyle

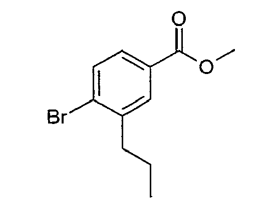

Dans un réacteur de 6 litres, équipé d'un réfrigérant, d'une agitation mécanique centrale, d'un thermomètre et d'une ampoule de coulée, introduire sous azote 141 g (0,46 moles) de 4-bromo-3-bromomethyl benzoate de méthyle, 4 litres de tetrahydrofuranne et 8,9 g (0,045 moles) d'iodure de cuivre(I). Refroidir le milieu à - 40°C à l'aide d'un bain d'acétonitrile et de carboglace. Additionner 168cc d'une solution 3M de bromure d'éthyle magnésium dans l'éther diéthylique, puis laisser remonter la température réactionnelle vers 0°C. Après deux heures de réaction, hydrolyser le milieu réactionnel avec 1 litre d'une solution 2,5M de chlorure d'ammonium. Séparer les phases et extraire la phase aqueuse avec 0,7 litre de chlorure de méthylène. Réunir les phases organiques et évaporer les solvants. Verser de l'heptane sur le produit obtenu et filtrer l'insoluble qui s'est formé. Evaporer l'heptane et purifier le produit obtenu par chromatographie sur gel de silice (heptane/acétate d'éthyle=95/5). On obtient 51 g de 4-bromo-3-propyl benzoate de méthyle sous forme d'une huile incolore. (Rdt=43%)
RMN 1 H (CDCl3 ; 400MHz) : 1,01 (t ; 3H ; J₁=7,3 Hz) ; 1,68 (massif ; 2H) ; 2,76 (t ; 2H ; J₂=7,6 Hz); 3,92 (s ; 3H) ; 7,61 (d ; 1 H ; Jo=8,3Hz) ; 7,71 (d x d ; 1H ; Jo=8,3Hz et Jm=1,8Hz) ; 7,89 (d ; 1H ; Jm=1,8Hz)

### Etape 3 : 3-(4-bromo-3-propyl-phenyl)-pentan-3-ol)

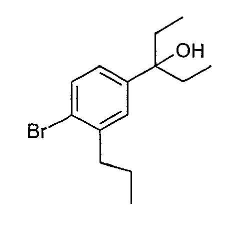

Dans un ballon tricol de 1 litre, équipé d'une agitation magnétique, d'un thermomètre et d'une ampoule de coulée, introduire sous azote 47 g (0,18 moles) de 4-bromo-3-propyl benzoate de méthyle en solution dans 250 mL de tetrahydrofuranne. Refroidir le milieu à -5°C à l'aide d'un bain de glace et de chlorure de sodium. Additionner 134cc d'une solution 3M de bromure d'éthyle magnésium dans l'éther diéthylique, puis laisser remonter la température réactionnelle sans dépasser 5°C. Après trois heures de réaction, hydrolyser le milieu réactionnel avec 170cc d'une solution 2,5M de chlorure d'ammonium. Allonger la phase organique avec 200cc d'acétate d'éthyle. Séparer les phases et extraire la phase aqueuse avec 2 X 200cc de chlorure de méthylène. Réunir les phases organiques, les sécher sur sulfate de sodium et évaporer les solvants. On obtient 49,8 g de 3-(4-bromo-3-propyl-phenyl)-pentan-3-ol sous forme d'une huile incolore. (Rdt=96%)
RMN 1 H (CDCl3 ; 400MHz) : 0,77 (t ; 6H ; J₂=7,4 Hz) ; 1,00 (t; 3H ; J₁=7,3 Hz) ; 1,68 (massif ; 2H) ; 1,83 (massif ; 4H) ; 2,73 (t ; 2H ; J₂=7,7 Hz) ; 7,05 (d x d ; 1H ; Jo=8,3Hz et Jm=2,2Hz) ; 7,24 (d ; 1 H ; Jm=2,2Hz) ; 7,48 (d ; 1 H ; Jo=8,3Hz)

### Etape 4 : Acide 4-(1-ethyl-1-hydroxy-propyl)-2-propyl-phenyl boronique

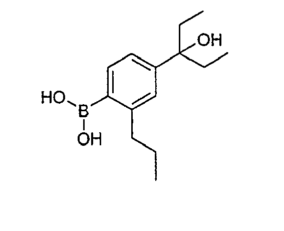

Dans un tricol, introduire sous azote 5 g de 3-(4-bromo-3-propyl-phenyl)-pentan-3-ol (17,5 mmoles) et 50cc de tetrahydrofuranne. Refroidir le milieu à -70°C et additionner goutte à goutte 17,5cc (43,7 mmoles) d'une solution 2,5M de n-butyllithium dans l'hexane en 30 minutes. Laisser environ 2h sous agitation à -70°C puis additionner goutte à goutte 10cc de triisopropyl borate (43,7 mmoles) en 20 minutes. Laisser sous agitation pendant 4h en laissant remonter la température vers -40°C. Hydrolyser le milieu avec une solution NH₄Cl saturée et extraire deux fois avec de l'acétate d'éthyle. Laver les phases organiques réunies avec une solution saturée de chlorure de sodium et évaporer les solvants sous vide. On obtient 5,4 g d'une huile incolore qu'on chromatographie sur gel de silice (heptane/acétate d'éthyle=3/1). On obtient 1,92 g d'acide 4-(1-ethyl-1-hydroxy-propyl)-2-propyl-phenyl boronique sous forme d'une huile très visqueuse (Rdt=44%)

### Exemple 2 :

### Etape 1 : 3-(4-Bromo-3-methyl-phenyl)-pentan-3-ol)

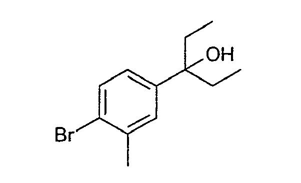

Dans un réacteur SVL de 4 litres, équipé d'une agitation magnétique, d'un thermomètre et d'une ampoule de coulée, introduire sous azote 198,7 g (0,867 moles) de 4-bromo-3-methyl benzoate de méthyle en solution dans 1 litre de tetrahydrofuranne. Refroidir le milieu à -5°C à l'aide d'un bain de glace et de chlorure de sodium. Additionner 636cc d'une solution 3M de bromure d'éthyle magnésium dans l'éther diéthylique, puis laisser remonter la température réactionnelle à température ambiante. Après quatre heures de réaction, hydrolyser le milieu réactionnel avec 2 litres d'une solution 1 N d'acide chlorhydrique. Après décantation et séparation, extraire la phase aqueuse avec 0,5 litre d'acétate d'éthyle. Laver les phases organiques réunies avec 0,4 litre d'eau (3X). Après séchage sur sulfate de sodium, le produit obtenu après évaporation des solvants est purifié par chromatographie sur gel de silice (heptane/acétate d'éthyle=95/5). On obtient 150,2 g de 3-(4-Bromo-3-methyl-phenyl)-pentan-3-ol sous forme d'une huile incolore. (Rdt=67%)
RMN 1H (CDCl3 ; 400MHz) : 0,79 (t; 6H ; J₂=7,4 Hz); 1,63 (s; 1H); 1,83 (massif ; 4H) ; 2,42 (s ; 3H) ; 7,05 (d x d ; 1 H ; Jo=8,3Hz et Jm=2,2Hz) ; 7,27 (d ; 1 H ; Jm=2,2Hz) ; 7,48 (d ; 1 H ; Jo=8,3Hz)

### Etape 2 : Acide 4-(1-ethyl-1-hydroxy-propyl)-2-methyl-phenyl boronique

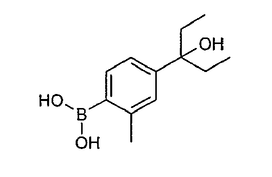

Dans un tricol, introduire sous azote 5 g de 3-(4-bromo-3-methyl-phenyl)-pentan-3-ol (19,4 mmoles) et 50cc de tetrahydrofuranne. Refroidir le milieu à -70°C et additionner goutte à goutte 23,3cc (58 mmoles) d'une solution 2,5M de n-butyllithium dans l'hexane en 30 minutes. Laisser environ 2h sous agitation à -70°C puis additionner goutte à goutte 13,5cc de triisopropyl borate (58 mmoles) en 20 minutes. Laisser sous agitation pendant 3h en laissant remonter la température vers -10°C. Hydrolyser le milieu avec une solution NH₄Cl saturée et extraire deux fois avec de l'acétate d'éthyle. Laver les phases organiques réunies avec une solution saturée de chlorure de sodium, les sécher sur sulfate de sodium. Evaporer les solvants sous vide. On obtient 5 g d'une huile incolore qu'on chromatographie sur gel de silice (heptane/acétate d'éthyle=9/1) pour donner l'acide 4-(1-ethyl-1-hydroxy-propyl)-2-methyl-phenyl boronique sous forme d'une huile très visqueuse (Rdt=71%).
RMN 1H (CDCl3 ; 400MHz) : 0,78 (t ; 3H ; J₂=7,4 Hz) ; 1,86 (massif ; 4H) ; 2,86 (s ; 3H) ; 7,31 (m ; 3H ;) ; 8,22 (d ; 1H ; Jo=8,3Hz)

### Exemple 3 - 3-[3-Propyl-4-(4,4.5,5-tetramethyl-[1,3,2]dioxaborolan-2-yl)-phenyl]-pentan-3-ol

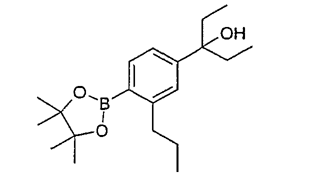

Dans un tricol, introduire sous azote 2 g de 3-(4-bromo-3-propyl-phenyl)-pentan-3-ol (7 mmoles) et 20cc de N,N-dimethylformamide. Ajouter 2,06 g (21 mmoles) d'acétate de potassium et 1,87 g (7,3 mmoles) de bis (pinacol) diborane. Additionner 0,229 g (0,28 mmoles) de dichloro [1,1'-ferrocenylbis(diphenylphosphane)] palladium (II) dichloromethane [Pd(dppf) Cl₂] et porter le milieu réactionnel au reflux pendant 2h30 Hydrolyser le milieu et extraire deux fois avec de l'acétate d'éthyle. Laver les phases organiques réunies avec une solution saturée de chlorure de sodium et évaporer les solvants sous vide. On obtient 3,6 g qu'on chromatographie sur gel de silice (heptane/acétate d'éthyle=8/2). On obtient 1,55 g de 3-[3-Propyl-4-(4,4,5,5-tetramethyl-[1,3,2]dioxaborolan-2-yl)-phenyl]-pentan-3-ol sous forme d'une huile très visqueuse (Rdt=76%)
RMN 1 H (CDCl3 ; 400MHz) : 0,76 (t ; 6H ; J₂=7,4 Hz) ; 0,95 (t ; 3H ; J₁=7,3 Hz) ; 1,36 (s ; 12H) ; 1,60 (massif; 2H) ; 1,67 (s ; 1 H) ; 1,84 (massif; 4H) ; 2,88 (t; 2H ; J₂=7,6 Hz) ; 7,18 (m ; 2H) ; 7,75 (d ; 1 H ; Jo=8,4Hz)

## Revendications

1. Composés disubstitués, dérivés de l'acide phényl-boronique, de formule générale (I) : 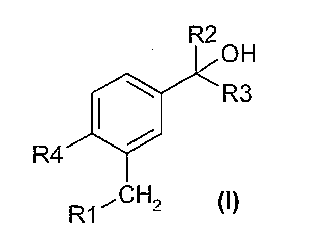 dans laquelle :
- R1 représente un atome d'hydrogène ou un radical alkyle de 1 à 4 atomes de carbone,
- R2 et R3, identiques ou différents représentent un radical alkyle de 1 à 4 atomes de carbone,
- R4 représente le radical -B(OH)₂ ou le radical de formule :

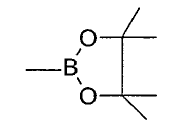

2. Composés selon la revendication 1, **caractérisés en ce que** les radicaux alkyles ayant de 1 à 4 atomes de carbone sont choisis parmi les radicaux méthyle, éthyle, propyle, isopropyle, butyle ou tertiobutyle, cyclopropyle.

3. Utilisation du composé de formule (1) suivante : 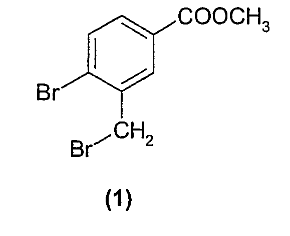 pour la préparation d'analogues non stéroidiens de vitamine D.

4. Composés de formule générale (2) suivante : 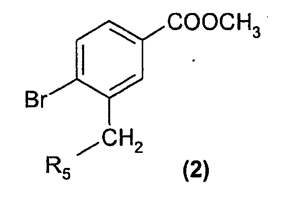 dans laquelle R5 représente un radical alkyle de 1 à 4 atomes de carbone.

5. Composés de formule générale (3) suivante : 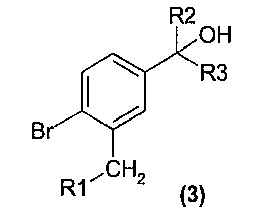 dans laquelle R1 représente un atome d'hydrogène ou un radical alkyle de 1 à 4 atomes de carbone, et R2 et R3, identiques ou différents représentent un groupement alkyle de 1 à 4 atomes de carbone.

6. Procédé de préparation des composés de formule générale (I) selon la revendication 1 avec R4 représente le radical B(OH)₂, à partir du 4-bromo-3-methyl benzoate de méthyle, **caractérisé en qu'**il comporte les étapes suivantes, les étapes a) et b) n'étant pas mises en oeuvre dans le cas particulier où R1 est un atome d'hydrogène :
a) bromation en position benzylique en présence d'un solvant pour obtenir le composé (1) 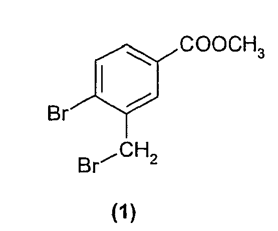
b) Substitution du brome introduit à l'étape (a) en position benzylique par une chaîne alkyle R5 comportant 1 à 4 atomes de carbones, pour obtenir le composé (2) selon la revendication 4 : 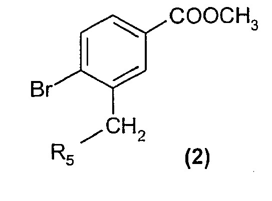
c) Transformation de la fonction ester des intermédiaires de formule générale (2) ou du 4-bromo-3-methyl benzoate de méthyle en fonction alcool tertiaire en utilisant au moins deux équivalents d'un organomagnésien de formule
R2-MgX
avec X représente un atome de Chlore ou de Brome
et R2 représente un radical méthyle, éthyle, propyle ou butyle
pour conduire au composé de formule générale (3) selon la revendication 5 ; 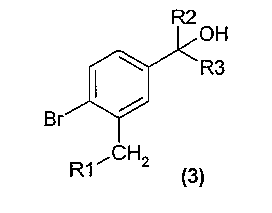
d) Transformation du bromure aromatique des intermédiaires de formule générale (3) en acide phényl-boronique de formule (I) en présence d'un solvant, d'une base et d'un trialkyle borate.

7. Procédé de préparation des composés de formule générale (I) selon la revendication 1, avec R4 représente le radical de formule 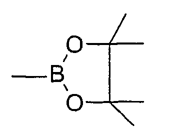 à partir du 4-bromo-3-methyl benzoate de méthyle, **caractérisé en qu'**il comporte les étapes suivantes, les étapes a) et b) n'étant pas mises en oeuvre dans le cas particulier où R1 est un atome d'hydrogène :
a) Bromation en position benzylique en présence d'un solvant pour obtenir le composé (1) ; 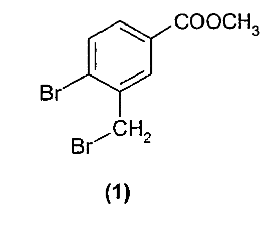
b) Substitution du brome introduit à l'étape (a) en position benzylique par une chaîne alkyle R5 comportant 1 à 4 atomes de carbones, pour obtenir le composé de formule générale (2) selon la revendication 4 : 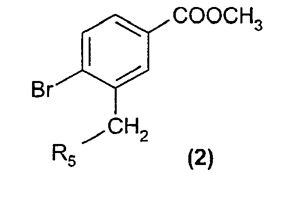
c) Transformation de la fonction ester des intermédiaires de formule générale (2) ou du 4-bromo-3-methyl benzoate de méthyle en fonction alcool tertiaire en utilisant au moins deux équivalents d'un organomagnésien de formule R2-MgX
avec X représente un atome de Chlore ou de Brome
et R2 représente un radical méthyle, éthyle, propyle ou butyle
pour conduire au composé de formule générale (3) selon la revendication 5 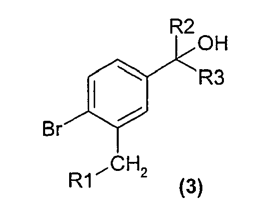
d) Transformation du bromure aromatique en aryl boronate en en présence d'un solvant, d'un catalyseur, d'une base et d'un tétra alkyle de bore pour obtenir le composé de formule générale (I).

8. Procédé selon la revendication 6 ou 7, **caractérisé en ce que** l'étape (a) s'effectue en présence :
- d'au moins un agent de bromation choisi parmi le brome, la N-bromosuccinimide, le bromate de sodium ou la 1,3-dibromo-5,5-dimethylhydantoïne ;
- d'un solvant choisi parmi les solvants chlorés ou les éthers.

9. Procédé selon l'une quelconque des revendications 6 à 8, **caractérisé en ce que** l'étape (a) s'effectue avec la N-bromosuccunimide en présence de dichlorométhane.

10. Procédé selon l'une quelconque des revendications 6 à 9, **caractérisé en ce qu'**on utilise du péroxyde de benzoyle comme initiateur radicalaire de la réaction de l'étape (a), la réaction radicalaire étant activée par irradiation du milieu réactionnel avec une lampe de 1000 watts.

11. Procédé selon l'une quelconque des revendications 6 à 10, **caractérisé en ce que** la réaction de substitution de l'étape b) est réalisée en présence d'un réactif organométallique R'1-M dans lequel M représente un atome de métal comme le magnésium, le cuivre ou encore le zinc.

12. Procédé selon la revendication 11, **caractérisé en ce que** le réactif organométallique est l'iodure de cuivre utilisé en présence d'un organomagnésien R'1-MgX dans lequel X représente un atome de brome, de Chlore ou d'iode.

13. Procédé selon l'une quelconque des revendications 11 à 12, **caractérisé en ce que** la réaction de l'étape (b) s'effectue en présence d'un solvant éthéré, tel le tétrahydrofuranne.

14. Procédé selon l'une quelconque des revendications 11 à 13, **caractérisé en ce que** la réaction de l'étape (b) s'effectue entre -40° et -20°C.

15. Procédé selon l'une quelconque des revendications 6 à 14, **caractérisé en ce que** la réaction effectuée à l'étape c) est réalisée en présence d'un solvant éthéré, tel le tétrahydrofuranne à une température située entre -10° et +10°C.

16. Procédé selon l'une quelconque des revendications 6 à 15, **caractérisé en ce que** la réaction effectuée à l'étape d) est réalisée en présence d'un solvant éthéré, d'une base forte et d'un trialkyl borate.

17. Procédé selon la revendication 16, **caractérisé en ce que** la réaction de l'étape (d) est réalisée à une température comprise entre -78°C et -40°C, en présence de tétrahydrofuranne, de 2,5 à 3,5 équivalents de butyllithium, et de 2 à 4 équivalents de triisopropyl borate.

18. Procédé selon l'une quelconque des revendications 7 à 15 précédentes **caractérisée en ce que** la réaction effectuée à l'étape d) est réalisée en présence :
- d'un solvant choisi parmi le DMF, le tetrahydrofuranne, le DMSO ou le dioxanne ;
- d'un catalyseur au palladium choisi parmi le dichloro [1,1'-ferrocenylbis(diphenylphosphane)] palladium (II) dichloromethane et le Pd(dppf) Cl₂ ;
- d'une base, choisi parmi l'acétate de potassium et la triethylamine ;
- d'un tetra alkoxyde de bore, choisi parmi le pinacol-borane et le pinacol-diborane.

19. Utilisation des composés de formule générale (I) : 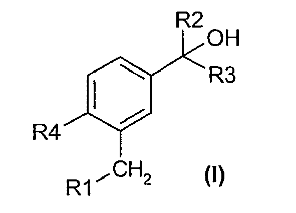 dans laquelle :
- R1 représente un atome d'hydrogène ou un radical alkyle de 1 à 4 atomes de carbone,
- R2 et R3, identiques ou différents représentent un radical alkyle de 1 à 4 atomes de carbone,
- R4 représente le radical -B(OH)₂ ou le radical de formule : 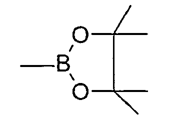 pour la préparation d'analogues non stéroidiens de vitamine D.

20. Utilisation selon la revendication 19, **caractérisée en ce que** les analogues non stéroidiens de vitamine D sont des composés de formule (IV) suivante : 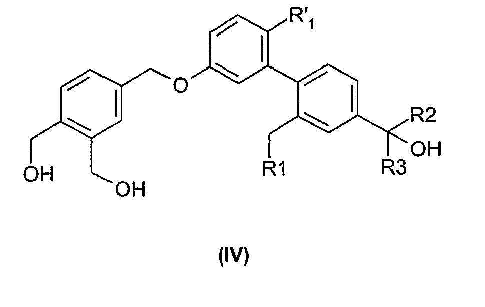 dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones.

21. Procédé de synthèse d'analogues non stéroïdiens de la vitamine D, **caractérisé en ce qu'**au moins une étape utilise les composés de formule générale (I) suivante : 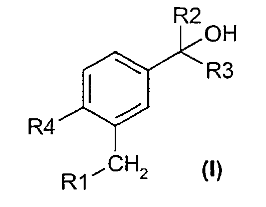 dans laquelle :
- R1 représente un atome d'hydrogène ou un radical alkyle de 1 à 4 atomes de carbone,
- R2 et R3, identiques ou différents représentent un radical alkyle de 1 à 4 atomes de carbone,
- R4 représente le radical -B(OH)₂ ou le radical de formule :

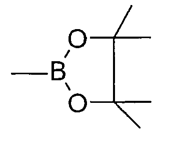

22. Procédé selon la revendication 21, **caractérisé en ce que** les analogues non stéroidiens de la vitamine D sont les composés de formule générale (IV) suivante : 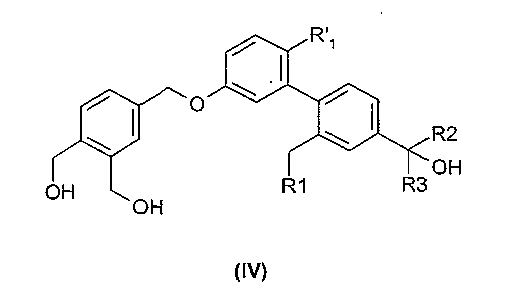 dans laquelle :
- R₁ représente un atome d'hydrogène ou un radical alkyle de 1 à 4 atomes de carbone ;
- R₂ et R₃ identiques ou différents représentent un radical alkyle de 1 à 4 atomes de carbone ;
- R'₁ représente un radical alkyle de 1 à 4 atomes de carbones.

23. Procédé selon la revendication 22, **caractérisé en ce qu'**il comporte les étapes suivantes :
a) Réaction de couplage de type Suzuki entre les composés de formule générale (I) 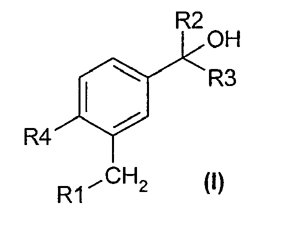 dans laquelle :
- R1 représente un atome d'hydrogène ou un radical alkyle de 1 à 4 atomes de carbone,
- R2 et R3, identiques ou différents représentent un radical alkyle de 1 à 4 atomes de carbone,
- R4 représente le radical -B(OH)₂ ou le radical de formule : 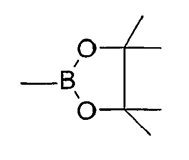 et un bromure d'aryle de formule générale (II) : 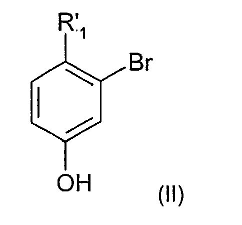
dans laquelle :
- R'₁ représente un radical alkyle de 1 à 4 atomes de carbones, pour obtenir le produit de formule générale (10) : 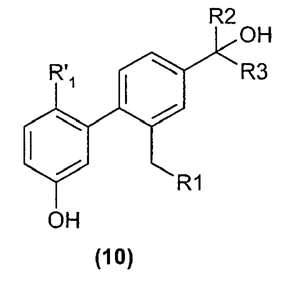
dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones ;
b) Le produit de la réaction de Suzuki, de formule générale (10), est condensé sur le bromure benzylique (11) en présence d'une base, tel le carbonate de potassium 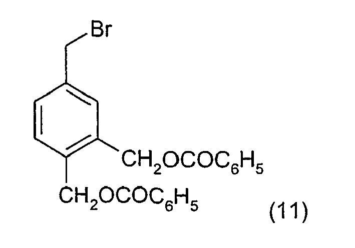
c) Libération des fonctions alcool du composé (11) pour conduire au composé (IV).

24. Composés de formule générale (10) suivante : 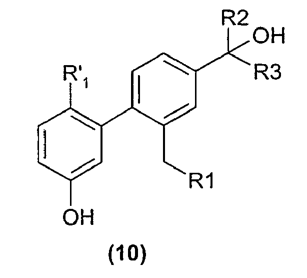 dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones.

25. Utilisation des composés de formule générale (10) selon la revendication 24 pour la préparation d'analogues non stéroidiens de vitamine D de formule générale (IV) : 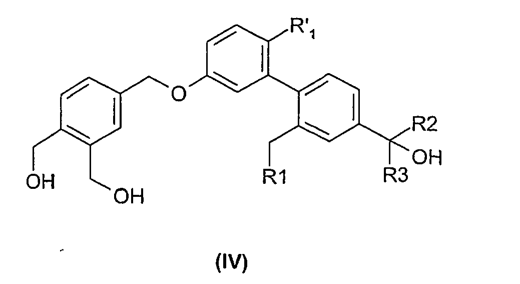 dans laquelle :
- R₁ représente un atome d'hydrogène ou un radical alkyle de 1 à 4 atomes de carbone
- R₂ et R₃ identiques ou différents représentent un radical alkyle de 1 à 4 atomes de carbone
- R'₁ représente un radical alkyle de 1 à 4 atomes de carbones.

## Claims

1. Disubstituted compounds which are derivatives of phenylboronic acid and are of general formula (I): 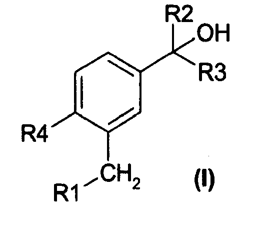 in which:
- R1 represents a hydrogen atom or an alkyl radical of 1 to 4 carbon atoms,
- R2 and R3, which are identical or different, represent an alkyl radical of 1 to 4 carbon atoms, and
- R4 represents the -B(OH)₂ radical or the radical of formula:

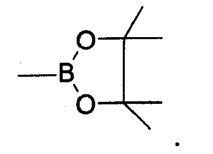

2. Compounds according to Claim 1, **characterized in that** the alkyl radicals having 1 to 4 carbon atoms are selected from methyl, ethyl, propyl, isopropyl, butyl or tert-butyl and cyclopropyl radicals.

3. Use of the compound of formula (1) below: 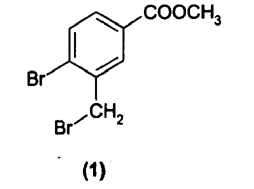 for preparing non-steroidal vitamin D analogues.

4. Compounds of general formula (2) below: 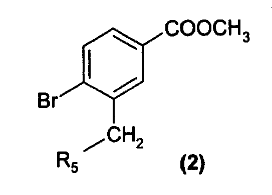 in which R₅ represents an alkyl radical of 1 to 4 carbon atoms.

5. Compounds of general formula (3) below: 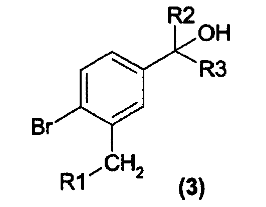 in which R1 represents a hydrogen atom or an alkyl radical of 1 to 4 carbon atoms and R2 and R3, which are identical or different, represent an alkyl group of 1 to 4 carbon atoms.

6. Process for preparing compounds of general formula (I) according to Claim 1 where R4 represents the B(OH)2 radical, from methyl 4-bromo-3-methylbenzoate, **characterized in that** it comprises the following steps, steps a) and b) not being implemented in the specific case where R1 is a hydrogen atom:
a) bromination in the benzylic position in the presence of a solvent, to give the compound (1) 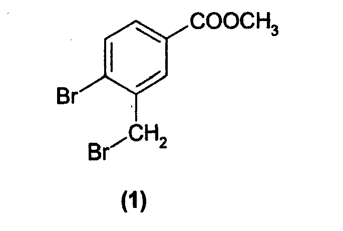
b) substitution of the bromine introduced in step a) in the benzylic position by an alkyl chain R₅ containing 1 to 4 carbon atoms, to give the compound (2) according to Claim 4: 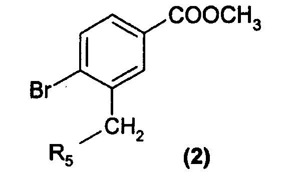
c) conversion of the ester function of the intermediates of general formula (2) or of methyl 4-bromo-3-methylbenzoate into a tertiary alcohol function, using at least two equivalents of an organomagnesium compound of formula R2-MgX
where X represents a chlorine or bromine atom
and R2 represents a methyl, ethyl, propyl or butyl radical,
to give the compound of general formula (3) according to Claim 5: 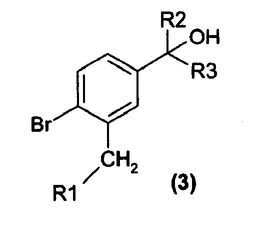
d) conversion of the aromatic bromide of the intermediates of general formula (3) into phenylboronic acid of formula (I) in the presence of a solvent, a base and a trialkyl borate.

7. Process for preparing compounds of general formula (I) according to Claim 1 where R4 represents the radical of formula 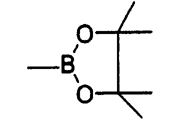 from methyl 4-bromo-3-methylbenzoate, **characterized in that** it comprises the following steps, steps a) and b) not being implemented in the specific case where R1 is a hydrogen atom:
a) bromination in the benzylic position in the presence of a solvent, to give the compound (1): 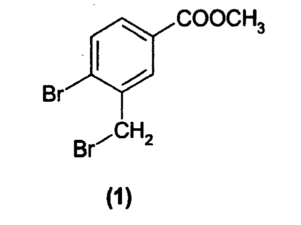
b) substitution of the bromine introduced in step a) in the benzylic position by an alkyl chain R₅ containing 1 to 4 carbon atoms, to give the compound of general formula (2) according to Claim 4: 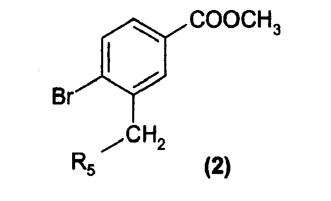
c) conversion of the ester function of the intermediates of general formula (2) or of methyl 4-bromo-3-methylbenzoate into a tertiary alcohol function, using at least two equivalents of an organomagnesium compound of formula R2-MgX
where X represents a chlorine or bromine atom
and R2 represents a methyl, ethyl, propyl or butyl radical
to give the compound of general formula (3) according to Claim 5: 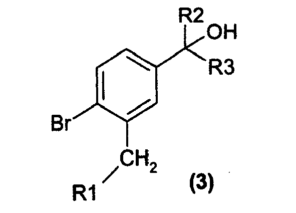
d) conversion of the aromatic bromide into aryl boronate in the presence of a solvent, a catalyst, a base and a boron tetraalkyl, to give the compound of general formula (I).

8. Process according to Claim 6 or 7, **characterized in that** step a) is carried out in the presence of:
- at least one brominating agent selected from bromine, N-bromosuccinimide, sodium bromate and 1,3-dibromo-5,5-dimethylhydantoin;
- a solvent selected from chlorinated solvents and ethers.

9. Process according to any one of Claims 6 to 8, **characterized in that** step a) is carried out with N-bromosuccinimide in the presence of dichloromethane.

10. Process according to any one of Claims 6 to 9, **characterized in that** benzoyl peroxide is used as free-radical initiator of the reaction of step a), the free-radical reaction being activated by irradiation of the reaction mixture with a 1000 watt lamp.

11. Process according to any one of Claims 6 to 10, **characterized in that** the substitution reaction of step b) is carried out in the presence of an organometallic reagent R'1-M in which M represents a metal atom such as magnesium, copper or else zinc.

12. Process according to Claim 11, **characterized in that** the organometallic reagent is copper iodide, which is used in the presence of an organomagnesium compound R'1-MgX in which X represents a bromine, chlorine or iodine atom.

13. Process according to either of Claims 11 and 12, **characterized in that** the reaction of step b) is carried out in the presence of an ethereal solvent, such as tetrahydrofuran.

14. Process according to any one of Claims 11 to 13, **characterized in that** the reaction of step b) is carried out at between -40°C and -20°C.

15. Process according to any one of Claims 6 to 14, **characterized in that** the reaction performed in step c) is carried out in the presence of an ethereal solvent, such as tetrahydrofuran, at a temperature situated between -10° and +10°C.

16. Process according to any one of Claims 6 to 15, **characterized in that** the reaction performed in step d) is carried out in the presence of an ethereal solvent, a strong base and a trialkyl borate.

17. Process according to Claim 16, **characterized in that** the reaction of step d) is carried out at a temperature of between -78°C and -40°C in the presence of tetrahydrofuran, from 2.5 to 3.5 equivalents of butyllithium and from 2 to 4 equivalents of triisopropyl borate.

18. Process according to any one of Claims 7 to 15 above, **characterized in that** the reaction performed in step d) is carried out in the presence of:
- a solvent selected from DMF, tetrahydrofuran, DMSO and dioxane;
- a palladium catalyst selected from dichloro[1,1'-ferrocenylbis(diphenylphosphane)]palladium(II) dichloromethane and Pd(dppf) Cl₂;
- a base selected from potassium acetate and triethylamine;
- a boron tetraalkoxide selected from pinacolborane and pinacoldiborane.

19. Use of the compounds of general formula (I): 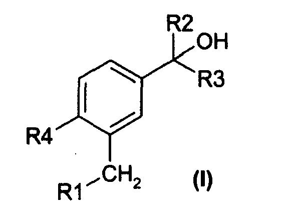 in which:
- R1 represents a hydrogen atom or an alkyl radical of 1 to 4 carbon atoms,
- R2 and R3, which are identical or different, represent an alkyl radical of 1 to 4 carbon atoms, and
- R4 represents the -B(OH)₂ radical or the radical of formula: 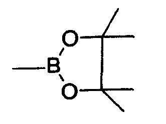 for preparing non-steroidal vitamin D analogues.

20. Use according to Claim 19, **characterized in that** the non-steroidal vitamin D analogues are compounds of formula (IV) below: 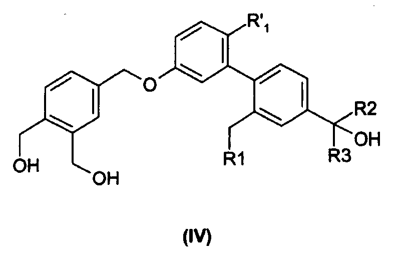 in which:
- R1 represents a hydrogen atom or an alkyl radical of 1 to 4 carbon atoms,
- R2 and R3, which are identical or different, represent an alkyl radical of 1 to 4 carbon atoms, and
- R'₁ represents an alkyl radical of 1 to 4 carbon atoms.

21. Process for synthesizing non-steroidal vitamin D analogues, **characterized in that** at least one step uses the compounds of general formula (I) below: 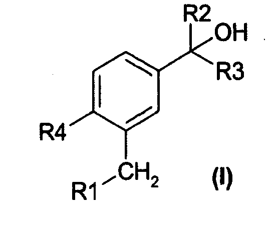 in which:
- R1 represents a hydrogen atom or an alkyl radical of 1 to 4 carbon atoms,
- R2 and R3, which are identical or different, represent an alkyl radical of 1 to 4 carbon atoms, and
- R4 represents the -B(OH)₂ radical or the radical of formula:

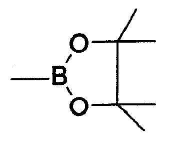

22. Process according to Claim 21, **characterized in that** the non-steroidal vitamin D analogues are compounds of general formula (IV) below: 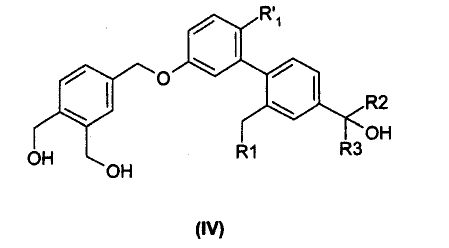 in which:
- R1 represents a hydrogen atom or an alkyl radical of 1 to 4 carbon atoms,
- R2 and R3, which are identical or different, represent an alkyl radical of 1 to 4 carbon atoms, and
- R'₁ represents an alkyl radical of 1 to 4 carbon atoms.

23. Process according to Claim 22, **characterized in that** it comprises the following steps:
a) a Suzuki coupling reaction between the compounds of general formula (I) 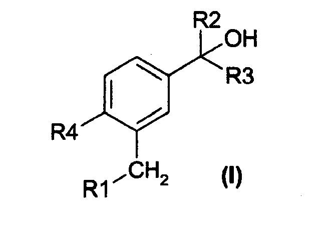 in which:
- R1 represents a hydrogen atom or an alkyl radical of 1 to 4 carbon atoms,
- R2 and R3, which are identical or different, represent an alkyl radical of 1 to 4 carbon atoms, and
- R4 represents the -B(OH)₂ radical or the radical of formula: 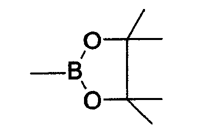 and an aryl bromide of general formula (II): 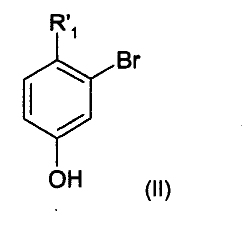
in which:
- R'₁ represents an alkyl radical of 1 to 4 carbon atoms,
to give the product of general formula (10): 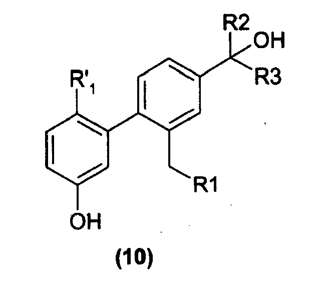
in which:
- R1 represents a hydrogen atom or an alkyl radical of 1 to 4 carbon atoms,
- R2 and R3, which are identical or different, represent an alkyl radical of 1 to 4 carbon atoms, and
- R'₁ represents an alkyl radical of 1 to 4 carbon atoms;
b) the Suzuki reaction product, of general formula (10), is fused with the benzyl bromide (11) in the presence of a base, such as potassium carbonate: 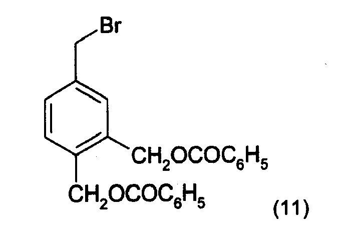
c) liberation of the alcohol functions of the compound (11) to give the compound (IV).

24. Compounds of general formula (10) below: 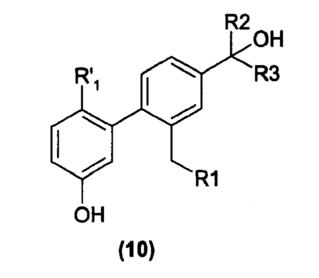 in which:
- R1 represents a hydrogen atom or an alkyl radical of 1 to 4 carbon atoms,
- R2 and R3, which are identical or different, represent an alkyl radical of 1 to 4 carbon atoms, and
- R'₁ represents an alkyl radical of 1 to 4 carbon atoms.

25. Use of compounds of general formula (10) according to Claim 24 for preparing non-steroidal vitamin D analogues of general formula (IV): 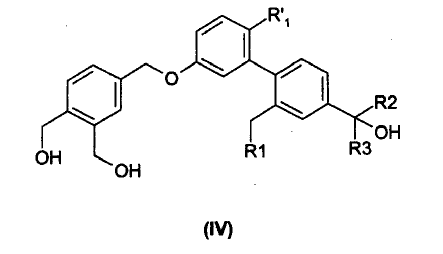 in which:
- R1 represents a hydrogen atom or an alkyl radical of 1 to 4 carbon atoms,
- R2 and R3, which are identical or different, represent an alkyl radical of 1 to 4 carbon atoms, and
- R'₁ represents an alkyl radical of 1 to 4 carbon atoms.

## Patentansprüche

1. Disubstituierte, von Phenylboronsäure abgeleitete Verbindungen der allgemeinen Formel (I): 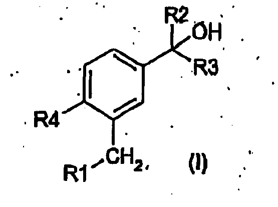 in der Formel:
- R1 bedeutet ein Wasserstoffatom oder eine Alkylgruppe mit 1 bis 4 Kohlenstoffatomen,
- die Gruppen R2 und R3, die gleich oder verschieden sind, bedeuten eine Alkylgruppe mit 1 bis 4 Kohlenstoffatomen,
- R4 bedeutet die Gruppe -B(OH)₂ oder die Gruppe der folgenden Formel:

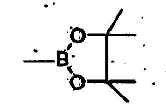

2. Verbindungen nach Anspruch 1, **dadurch gekennzeichnet, dass** die Alkylgruppen mit 1 bis 4 Kohlenstoffatomen unter den Gruppen Methyl, Ethyl, Propyl, Isopropyl, Butyl oder *tert*-Butyl, Cyclopropyl ausgewählt sind.

3. Verwendung der Verbindung der folgenden Formel (1): 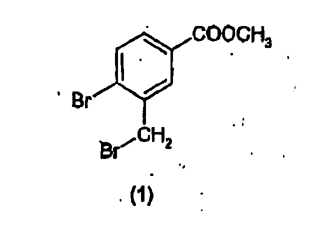 für die Herstellung von nichtsteroidalen Vitamin D-Analoga.

4. Verbindungen der folgenden allgemeinen Formel (2): 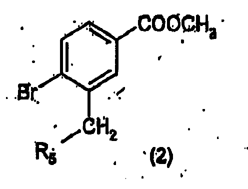 in der R₅ eine Alkylgruppe mit 1 bis 4 Kohlenstoffatomen bedeutet.

5. Verbindungen der folgenden allgemeinen Formel (3): 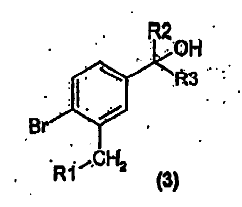 worin R1 ein Wasserstoffatom oder eine Alkylgruppe mit 1 bis 4 Kohlenstoffatomen bedeutet und die Gruppen R2 und R3, die gleich oder verschieden sind, eine Alkylgruppe mit 1 bis 4 Kohlenstoffatomen bedeuten.

6. Verfahren zur Herstellung von Verbindungen der allgemeinen Formel (I) nach Anspruch 1, wobei R4 die Gruppe B(OH)₂ bedeutet, ausgehend von Methyl-4-brom-3-methyl-benzoat, **dadurch gekennzeichnet, dass** es die folgenden Schritte umfasst, wobei die Schritte a) und b) in dem speziellen Fall, wenn R1 ein Wasserstoffatom bedeutet, nicht durchgeführt werden:
a) Bromierung in der Benzylstellung in Gegenwart eines Lösungsmittels, um die Verbindung (1) herzustellen: 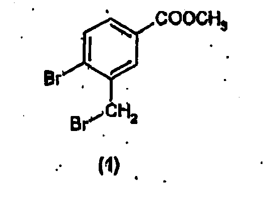
b) Substitution des in Schritt a) in Benzylstellung eingeführten Bromatoms durch eine Alkylkette R5, die 1 bis 4 Kohlenstoffatome aufweist, um die Verbindung (2) nach Anspruch 4 zu bilden; 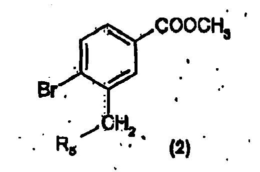
c) Transformation der Esterfunktion der Zwischenprodukte der allgemeinen Formel (2) oder des Methyl-4-brom-3-methyl-benzoats in eine tertiäre Alkoholfunktion unter Verwendung von zwei Äquivalenten einer Magnesium-organischen Verbindung der Formel R2-MgX,
wobei X ein Chloratom oder ein Bromatom bedeutet und
R2 eine Methylgruppe, Ethylgruppe, Propylgruppe oder Butylgruppe ist,
zur Bildung einer Verbindung der allgemeinen Formel (3) nach Anspruch 5; 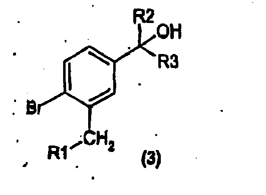
d) Transformation des aromatischen Bromids der Zwischenprodukte der allgemeinen Formel (3) in die Phenylboronsäure der Formel (I) in Gegenwart eines Lösungsmittels, einer Base und eines Trialkylborats.

7. Verfahren zur Herstellung von Verbindungen der allgemeinen Formel (I) nach Anspruch 1, wobei R4 die Gruppe der folgenden Formel bedeutet; 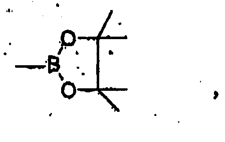 ausgehend von Methyl-4-brom-3-methylbenzoat, **dadurch gekennzeichnet, dass** es die folgenden Schritte umfasst, wobei die Schritte a) und b) in dem speziellen Fall, wenn R1 ein Wasserstoffatom bedeutet, nicht durchgeführt werden:
a) Bromierung in der Benzylstellung in Gegenwart eines Lösungsmittels, um die Verbindung (1) herzustellen: 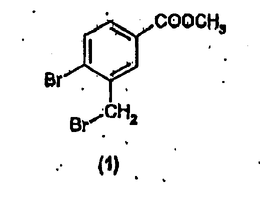
b) Substitution des in Schritt a) in Benzylstellung eingeführten Bromatoms durch eine Alkylkette R5, die 1 bis 4 Kohlenstoffatome aufweist, um die Verbindung (2) nach Anspruch 4 zu bilden: 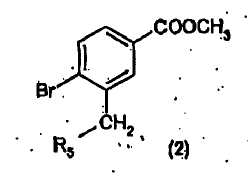
c) Transformation der Esterfunktion der Zwischenprodukte der allgemeinen Formel (2) oder des Methyl-4-brom-3-methyl-benzoats in eine tertiäre Alkoholfunktion unter Verwendung von zwei Äquivalenten einer Magnesium-organischen Verbindung der Formel R2-MgX,
wobei X ein Chloratom oder ein Bromatom bedeutet und
R2 eine Methylgruppe, Ethylgruppe, Propylgruppe oder Butylgruppe ist,
zur Bildung einer Verbindung der allgemeinen Formel (3) nach Anspruch 5; 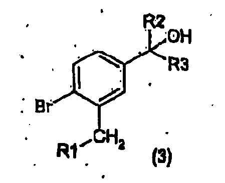
d) Transformation des aromatischen Bromids in ein Arylboronat in Gegenwart eines Lösungsmittels, eines Katalysators, einer Base und eines Bortetraalkoxids, um die Verbindung der allgemeinen Formel (I) zu bilden.

8. Verfahren nach Anspruch 6 oder 7, **dadurch gekennzeichnet, dass** Schritt a) durchgeführt wird in Gegenwart:
- mindestens eines Bromierungsmittels, das unter Brom, N-Bromsuccinimid, Natriumbromat oder 1,3-Dibrom-5,5-dimethylhydantoin ausgewählt ist;
- eines Lösungsmittels, das unter den chlorierten Lösungsmitteln oder Ethern ausgewählt ist.

9. Verfahren nach einem der Ansprüche 6 bis 8, **dadurch gekennzeichnet, dass** Schritt a) mit N-Bromsuccinimid in Gegenwart von Dichlormethan durchgeführt wird. '

10. Verfahren nach einem der Ansprüche 6 bis 9, **dadurch gekennzeichnet, dass** als Radikalstarter für die Reaktion des Schrittes
a) Benzoylperoxid verwendet wird, wobei die radikalische Reaktion durch Bestrahlung des Reaktionsmediums mit einer Lampe von 1 000 Watt aktiviert wird.

11. Verfahren nach einem der Ansprüche 6 bis 10, **dadurch gekennzeichnet, dass** die Substitution des Schrittes b) in Gegenwart eines Metall-organischen Reaktanten R'1-M erfolgt, wobei M ein Metallatom bedeutet, wie Magnesium, Kupfer oder auch Zink.

12. Verfahren nach Anspruch 11, **dadurch gekennzeichnet, dass** es sich bei dem Metall-organischen Reaktanten um Kupferiodid handelt, das in Gegenwart einer Magnesium-organischen Verbindung R'1-MgX verwendet wird, wobei X ein Bromatom, Chloratom oder Iodatom bedeutet.

13. Verfahren nach einem der Ansprüche 11 bis 12, **dadurch gekennzeichnet, dass** die Reaktion des Schrittes b) in Gegenwart eines Etherlösungsmittels wie Tetrahydrofuran durchgeführt wird.

14. Verfahren nach einem der Ansprüche 11 bis 13, **dadurch gekennzeichnet, dass** die Reaktion des Schrittes b) bei -40 bis
- 20 °C erfolgt.

15. Verfahren nach einem der Ansprüche 6 bis 14, **dadurch gekennzeichnet, dass** die in Schritt c) durchgeführte Umsetzung in Gegenwart eines Etherlösungsmittels, wie Tetrahydrofuran, bei einer Temperatur im Bereich von -10 bis +10 °C durchgeführt wird.

16. Verfahren nach einem der Ansprüche 6 bis 15, **dadurch gekennzeichnet, dass** die in Schritt d) durchgeführte Umsetzung in Gegenwart eines Etherlösungsmittels, einer starken Base und eines Trialkylborats durchgeführt wird.

17. Verfahren nach Anspruch 16, **dadurch gekennzeichnet, dass** die Umsetzung des Schrittes d) bei einer Temperatur im Bereich von -78 bis -40 °C in Gegenwart von Tetrahydrofuran, 2,5 bis 3,5 Äquivalenten Butyllithium und 2 bis 4 Äquivalenten Trüsobutylborat durchgeführt wird.

18. Verfahren nach einem der vorhergehenden Ansprüche 7 bis 15, **dadurch gekennzeichnet, dass** die in Schritt d) durchgeführte Umsetzung durchgeführt wird in Gegenwart:
- eines Lösungsmittels, das unter DMF, Tetrahydrofuran, DMSO oder Dioxan ausgewählt ist;
- eines Palladiumkatalysators, der unter Dichlor- [1,1'-ferrocenylbis(diphenylphosphan)]-palladium(II)-dichlormethan und Pd(dppf)Cl₂ ausgewählt ist;
- einer Base, die unter Kaliumacetat und Triethylamin ausgewählt ist;
- eines Bortetraalkoxids, das unter Pinacolboran und Pinacoldiboran ausgewählt ist.

19. Verwendung der Verbindungen der allgemeinen Formel (I): 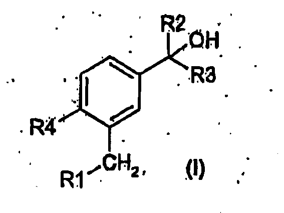 wobei in der Formel:
- R1 ein Wasserstoffatom oder eine Alkylgruppe mit 1 bis 4 Kohlenstoffatomen bedeutet,
- die Gruppen R2 und R3, die gleich oder verschieden sind, eine Alkylgruppe mit 1 bis 4 Kohlenstoffatomen bedeuten,
- R4 die Gruppe B(OH)₂ oder die Gruppe der folgenden Formel bedeutet: 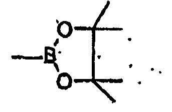 für die Herstellung von nichtsteroidalen Vitamin D-Analoga.

20. Verwendung nach Anspruch 19, **dadurch gekennzeichnet, dass** die nichtsteroidalen Vitamin D-Analoga Verbindungen der folgenden Formel (IV) sind: 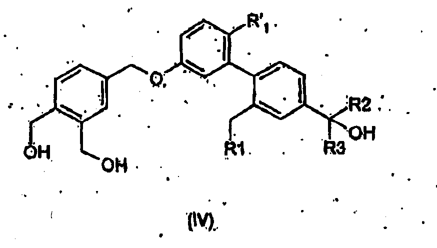 in der Formel:
- R₁ bedeutet ein Wasserstoffatom oder eine Alkylgruppe mit 1 bis 4 Kohlenstoffatomen,
- die Gruppen R₂ und R₃, die gleich oder verschieden sind, bedeuten eine Alkylgruppe mit 1 bis 4 Kohlenstoffatomen,
- R'₁ bedeutet eine Alkylgruppe mit 1 bis 4 Kohlenstoffatomen.

21. Verfahren zur Synthese noch nichtsteroidalen Vitamin D-Analoga, **dadurch gekennzeichnet, dass** in zumindest einem Schritt Verbindungen der folgenden allgemeinen Formel (I) eingesetzt werden: 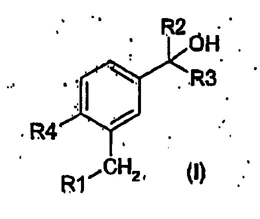 in der Formel:
- R1 bedeutet ein Wasserstoffatom oder eine Alkylgruppe mit 1 bis 4 Kohlenstoffatomen,
- die Gruppen R2 und R3, die gleich oder verschieden sind, bedeuten eine Alkylgruppe mit 1 bis 4 Kohlenstoffatomen,
- R4 bedeutet die Gruppe -B(OH)₂ oder die Gruppe der folgenden Formel:

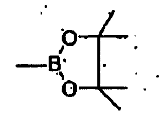

22. Verfahren nach Anspruch 21, **dadurch gekennzeichnet, dass** die nichtsteroidalen Vitamin D-Analoga Verbindungen der folgenden allgemeinen Formel (IV) sind: 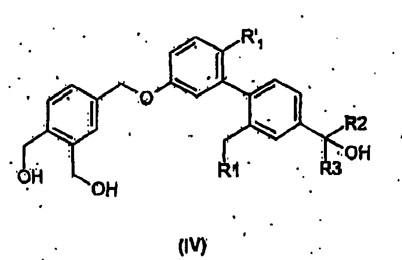 in der Formel:
- R₁ bedeutet ein Wasserstoffatom oder eine Alkylgruppe mit 1 bis 4 Kohlenstoffatomen,
- die Gruppen R₂ und R₃, die gleich oder verschieden sind, bedeuten eine Alkylgruppe mit 1 bis 4 Kohlenstoffatomen,
- R'₁ bedeutet eine Alkylgruppe mit 1 bis 4 Kohlenstoffatomen.

23. Verfahren nach Anspruch 22, **dadurch gekennzeichnet, dass** es die folgenden Schritte umfasst:
a) Kupplung vom Suzuki-Typ der Verbindungen der allgemeinen Formel (I): 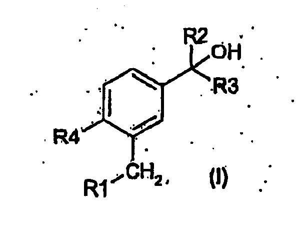 wobei in der Formel:
- R1 ein Wasserstoffatom oder eine Alkylgruppe mit 1 bis 4 Kohlenstoffatomen bedeutet,
- die Gruppen R2 und R3, die gleich oder verschieden sind, eine Alkylgruppe mit 1 bis 4 Kohlenstoffatomen bedeuten,
- R4 die Gruppe B(OH)₂ oder die Gruppe der folgenden Formel bedeutet; 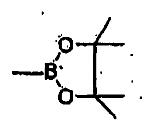 und eines Arylbromids der allgemeinen Formel (II): 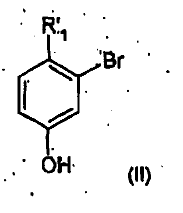 in der Formel:
- R'₁ bedeutet eine Alkylgruppe mit 1 bis 4 Kohlenstoffatomen,
zur Bildung des Produkts der allgemeinen Formel (10); 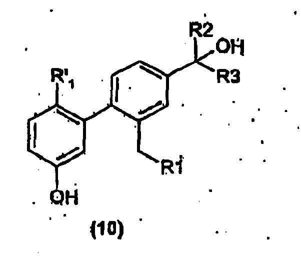 in der Formel:
- R₁ bedeutet ein Wasserstoffatom oder eine Alkylgruppe mit 1 bis 4 Kohlenstoffatomen,
- die Gruppen R₂ und R₃, die gleich oder verschieden sind, bedeuten eine Alkylgruppe mit 1 bis 4 Kohlenstoffatomen,
- R'₁ bedeutet eine Alkylgruppe mit 1 bis 4 Kohlenstoffatomen;
b) das Reaktionsprodukt der Suzuki-Kupplung der allgemeinen Formel (10) wird in Gegenwart einer Base, wie Kaliumcarbonat, mit dem Benzylbromid (11) kondensiert 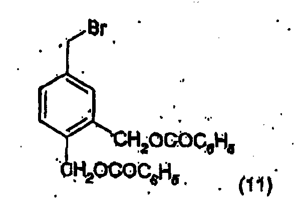
c) die Alkoholfunktionen der Verbindung (11) werden freigesetzt, wodurch die Verbindung (IV) gebildet wird.

24. Verbindungen der folgenden allgemeinen Formel (10): 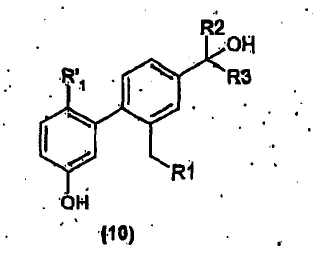 in der Formel:
- R₁ bedeutet ein Wasserstoffatom oder eine Alkylgruppe mit 1 bis 4 Kohlenstoffatomen,
- die Gruppen R₂ und R₃, die gleich oder verschieden sind, bedeuten eine Alkylgruppe mit 1 bis 4 Kohlenstoffatomen,
- R'₁ bedeutet eine Alkylgruppe mit 1 bis 4 Kohlenstoffatomen.

25. Verwendung der Verbindungen der allgemeinen Formel (10) nach Anspruch 24 für die Herstellung von nichtsteroidalen Vitamin D-Analoga der allgemeinen Formel (IV): 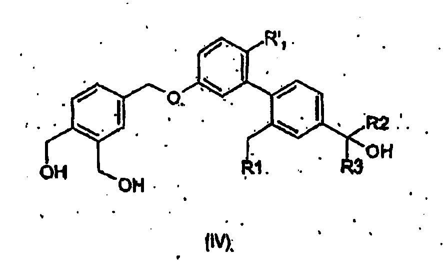 in der Formel:
- R₁ bedeutet ein Wasserstoffatom oder eine Alkylgruppe mit 1 bis 4 Kohlenstoffatomen,
- die Gruppen R₂ und R₃, die gleich oder verschieden sind, bedeuten eine Alkylgruppe mit 1 bis 4 Kohlenstoffatomen,
- R'₁ bedeutet eine Alkylgruppe mit 1 bis 4 Kohlenstoffatomen.
